# EUROPÄISCHE PATENTSCHRIFT

(11) **EP 4 025 918 B1**
(45) Veröffentlichungstag und Bekanntmachung des Hinweises auf die Patenterteilung: **17.12.2025**
(21) Anmeldenummer: 20761510.5
(22) Anmeldetag: 16.08.2020
(51) Int. Cl.: G01P 15/18, G01L 1/04, G01L 5/166, G01L 1/24

(54) **KRAFTMESSSCHEIBE SOWIE VORRICHTUNG ZUR BESTIMMUNG VON KRÄFTEN IM PIKO-NEWTON- BIS NANO-NEWTON-BEREICH**
FORCE MEASURING DISC AND DEVICE FOR DETERMINING FORCES IN THE PICO-NEWTON TO NANO-NEWTON RANGE
DISQUE DE MESURE DE FORCE ET DISPOSITIF DE DÉTERMINATION DES FORCES DANS LA RÉGION DE PICO-NEWTON À NANO-NEWTON

(30) Priorität: 02.09.2019 DE 102019123394
(43) Veröffentlichungstag der Anmeldung: 13.07.2022
(73) Patentinhaber: Universität Heidelberg, 69117 Heidelberg (DE)
(72) Erfinder: NEUMANN, Hendrikje, 24114 Kiel (DE); SELHUBER-UNKEL, Christine, 24107 Kiel (DE); QUANDT, Eckhard, 24226 Heikendorf (DE)
(74) Vertreter: Puschmann Borchert Kaiser Klettner Patentanwälte Partnerschaft mbB
(86) Internationale Anmeldenummer: PCT/DE2020/100712
(87) Internationale Veröffentlichungsnummer: WO 2021/043366

(56) Entgegenhaltungen:
- CN-B- 101 657 728
- US-A1- 2017 184 628
- JAYANETTI V C ET AL: "Design and simulation of a MEMS based dual axis capacitive accelerometer", 2015 MORATUWA ENGINEERING RESEARCH CONFERENCE (MERCON), IEEE, 7 April 2015 (2015-04-07), pages 194 - 198, XP032780691, [retrieved on 20150522], DOI: 10.1109/MERCON.2015.7112344
- KUDRLE T D ET AL: "Single-crystal silicon micromirror array with polysilicon flexures", SENSORS AND ACTUATORS A: PHYSICAL, ELSEVIER BV, NL, vol. 119, no. 2, 13 April 2005 (2005-04-13), pages 559 - 566, XP004852199, ISSN: 0924-4247, DOI: 10.1016/J.SNA.2004.10.038
- GENKI YOSHIKAWA ET AL: "Nanomechanical Membrane-type Surface Stress Sensor", NANO LETTERS, vol. 11, no. 3, 9 March 2011 (2011-03-09), US, pages 1044 - 1048, XP055506585, ISSN: 1530-6984, DOI: 10.1021/nl103901a
- SOLGAARD OLAV ET AL: "Optical MEMS: From Micromirrors to Complex Systems", JOURNAL OF MICROELECTROMECHANICAL SYSTEMS, IEEE SERVICE CENTER, US, vol. 23, no. 3, 1 June 2014 (2014-06-01), pages 517 - 538, XP011549786, ISSN: 1057-7157, [retrieved on 20140529], DOI: 10.1109/JMEMS.2014.2319266

## Beschreibung

Die Erfindung betrifft eine Vorrichtung zur Bestimmung von Kräften im Piko-Newton- bis Nano-Newton-Bereich mit wenigstens einer Kraftmessscheibe aufweisend eine ebene, regelmäßige Anordnung von Kraftmesszellen, wobei eine Kraftmesszelle gebildet ist in einem Loch in der Kraftmessscheibe, in dem genau ein von Federn freitragend gehaltenes Flächenelement angeordnet und parallel zur Kraftmessscheibe verlaufend ausgerichtet ist.

Die Erfindung könnte weiterhin auch als mechano-optischer submikronewton 3D Kraftmesser oder auch Mikrosensorsystem bezeichnet werden, was hier jedoch nicht beschränkend zu werten ist.

Dieses neuartige Mikrosensorsystem bzw. die Kraftmessscheibe ermöglicht 3D-Kraftmessungen im Pico- bis Mikronewton-Bereich insbesondere für mikrometergroße Klebeproben und lebende Systeme wie Zellen oder Bakterien.

In Zeiten schneller Entwicklung und wachsender Märkte im Bereich der Robotik, mit einem besonderen Augenmerk auf humanoide Roboter, der Hightech Prothetik und der personalisierten Medizin ist die Biomimetik natürlicher Materialien wie beispielsweise in Form künstlicher Haut zentrales Interesse in Forschung und Industrie. Um die Struktur-Funktions-Beziehungen in lebenden Systemen umfassend zu verstehen ist die umfangreiche Wissenserweiterung in Bezug auf den kleinsten lebenden Baustein, die Zelle, und seine Biomechanik Gegenstand weltweit laufender Forschungsprojekte. Dennoch gab es bis jetzt keine umfassende etablierte Methode, die 3D Zellkräfte zum Beispiel während des Zellwanderungsprozesses quantitativ messen kann.

Hier wurden in den letzten Jahren in vielseitigen Studien verschiedene Methoden entwickelt, um einen detaillierteren Einblick in die Zellmechanik zu erhalten. Ein besonderes Interesse galt der elektrochemischen Sensorik von Zellen durch äußere Reize, der so genannten Mechanotransduktion, die makroskopische Phänomene wie die Wundheilung durch Zellmigration verursacht. Um diese dynamischen Zellprozesse in zukünftigen Anwendungen zu manipulieren oder nachzuahmen, wurden Zellkräfte mit einer Vielzahl von Techniken wie Rasterkraftmikroskopie, mikroelektromechanischen Systemen (MEMS), der Verformung von Mikrosäulenarrays oder der Zugkraftmikroskopie und anderen erfasst. Keine der bisher etablierten Techniken ermöglichte jedoch eine quantitative Zellzugkraftmessung im Pico- bis Nanonewton-Bereich in drei Dimensionen auf einmal mittels einer kostengünstigen Einstellung.

Verschiedene Techniken zur Zellkraftmessung in ein, zwei und drei Dimensionen stehen zur Verfügung und sind teilweise bereits schon im Einsatz. Die aktuellen Methoden haben jedoch wenigstens einen großen Nachteil, wie beispielsweise einen begrenzten Kraftbereich, eine Begrenzung auf eine oder zwei Dimensionen, die Aufzeichnung nur qualitativer Messergebnisse, eine begrenzte Kraftauflösung aufgrund von Sensorelementdimensionen, die größer sind als die Zelldimension, und weiter eine hohe Komplexität oder Preisgestaltung der Anordnung.

Die Zellkrafterfassung über Polydimethylsiloxan (PDMS)-Mikrosäulen basiert beispielsweise auf einer deutlichen Verformung der Säulen in Abhängigkeit von der Zellkraftinteraktion. Mit dieser Methode können nur Kräfte in zwei Dimensionen erfasst werden. Weiterhin hängt die Homogenität der Elastizität über das gesamte PDMS-Substrat von der Homogenität des Polymerisationsverfahrens ab. Dieser Prozess kann über die gesamte Probenoberfläche variieren. Eine weitere weit verbreitete Technik ist die Zugkraftmikroskopie - transaction force microscopy (TFM), die auf der Verformung einer Hydrogelmembran durch Zellkräfte und einer rechnergestützten Trajektorieanalyse der Kraftwirkungen auf die Membran basiert. Nachteilig hierbei ist, dass die TFM sehr zeit- und kostenintensiv und empfindlich gegenüber Inhomogenitäten im Hydrogel ist. Darüber hinaus hängt die Auflösung dieser Technik von der Weichheit des Hydrogels ab und die Weichheit des Hydrogels beeinflusst das Zellverhalten. Daher verursachen Schwankungen des Verhaltens elastischer Materialien Unsicherheiten in den resultierenden Kraftdaten.

Mikroelektronische Systeme (MEMS), die zur Zellkrafterfassung verwendet werden, ermöglichen nur eine zweidimensionale Krafterfassung und qualitative Kraftdaten. Dreidimensionale Messungen erfordern die Kombination von mindestens zwei verschiedenen physikalischen Phänomenen wie Piezowiderstand und Kapazität, die zu einer Erhöhung der Dimension des Sensorelements führen.

Als relevante Patentdokumente, die sich genau derselben Aufgabenstellung widmen, werden die Druckschriften US 2017/322193 A1 und WO 2019/010234 A1 genannt. Die Herangehensweise beider Druckschriften entspricht etwa dem folgenden, nämlich dass die jeweils eine Vorrichtung zur Bestimmung von Kräften offenbart ist, insbesondere von sehr kleinen Kräften, die als Haftungs- und Traktionskräfte in lebenden (tierischen oder menschlichen) Zellen oder Zellclustern auftreten, wobei die Grundidee ist, diese Kräfte indirekt zu erfassen durch die Biegung oder Auslenkung vorbestimmter und - soweit möglich - vorkalibrierter Testelemente. Die Formänderungen der Testelemente sollen optisch erfasst und hiernach in von den Zellen ausgeübte Kräfte übersetzt werden, wobei die Kraftmessscheibe jedoch aus einem weichen, durch die Zellkräfte deformierbaren, dünnen Polymerfilm (z.B. PDMS) auf einer starren, transparenten Unterlage gebildet ist. Die auf der Vorderseite aufliegenden Zellen verformen den Polymerfilm und in den Film eingebettete optische Reflektoren (Mikrospiegel oder optische Kavitäten) werden dadurch elastisch ausgelenkt. Auch hier erfolgt die Messung durch optische Interferometrie von der Rückseite des Polymerfilms her. Hauptunterschied ist somit die Ausgestaltung der Kraftmessscheibe, die auch für sich genommen als Komponente der Gesamtvorrichtung eine kommerziell handelbare Ware sein dürfte.

In der Druckschrift "Nanomechanical Membrane-type Surface Stress Sensor", Genki Yoshikawa et al., Nano Letters, Bd. 11, Nr. 3, 9. März 2011 (2011-03-09), Seiten 1044-1048, XP055506585, US ISSN: 1530-6984, DOI: 10.1021/nl103901a wird ein membranartiger Oberflächenspannungssensor (MSS) beschrieben, der auf einer in einen Sensorchip integrierten piezoresistiven Auslesung basiert. Der MSS ist kein einfacher "Freischwinger", sondern besteht aus einer "Adsorbatmembran", die an vier piezoresistiven "Messbalken" aufgehängt ist und eine vollständige Wheatstone-Brücke bildet. Die gesamte durch den Analyten verursachte isotrope Oberflächenspannung auf der Membran wird als verstärkte einachsige Spannung effizient auf die piezoresistiven Balken übertragen.

Dabei ist festzustellen, dass es z.B. in der MEMS-Bauweise an sich bekannt ist, freitragende Flächen aufgehängt an Federstrukturen in Mäanderform aus einem zunächst monolithischen Materialblock heraus zu strukturieren oder auch alternativ mittels Sputter-Technologie aufzubauen. Mäander-Federn kommen insbesondere bei Mikrokraftsensoren zum Einsatz (vgl. DE 10 2010 012 701 A1).

Die Druckschrift US 2017/184628 A1 offenbart eine mikro-elektromechanische (MEMS) Vorrichtung umfassend ein Substrat, zwei erste Verankerungen, einen Rahmen und zwei elastische Elemente. Das Substrat ist mit einem Referenzpunkt versehen. Der Rahmen umgibt die beiden ersten Verankerungen, und jedes der elastischen Elemente verbindet die entsprechende erste Verankerung und den Rahmen. Jeder der ersten Anker ist in der Nähe der Mitte der MEMS-Vorrichtung angeordnet, um einen durch Verformung des Substrats verursachten Effekt zu verringern. Die MEMS-Vorrichtung kann für einen MEMS-Sensor mit einer drehbaren Masse, wie z. B. einen Drei-Achsen-Beschleunigungsmesser oder ein Magnetometer, verwendet werden, um die Prozessausbeute, Zuverlässigkeit und Messgenauigkeit zu verbessern.

Zudem ist aus der Druckschrift "Optical MEMS: From Micromirrors to Complex Systems", Solgaard Olav et. Al., Journal of Microeletromechanical Systems, IEEE Service Center, US, Bd. 23, Nr. 3, 11. Juni 2014 (2014-06-01), Seiten 517-538, XP011549786, ISSN: 1057-7157, DOI: 10.1109/JMEMS.2014.2319266 [gefunden am 2014-05-29] der Stand der Technik in Bezug auf optische mikro-elektromechanische Technologie (MEMS)-Sensoren bekannt. Die präzisen Abmessungen und die Ausrichtung von MEMS-Geräten in Verbindung mit der mechanischen Stabilität, die mit der Miniaturisierung einhergeht, machen optische MEMS-Sensoren für eine Vielzahl von anspruchsvollen Messungen geeignet.

Die Druckschrift CN 101 657 728 B beschreibt eine mikroelektromechanische Systemvorrichtung mit einem Substrat; mindestens einer auf dem Substrat angeordneten Halbleiterschicht; einem Schaltungsbereich mit mindestens einem Chip, der eine auf der mindestens einen Halbleiterschicht angeordnete Ansteuerungs-/Abtastschaltung enthält; einer an dem Substrat angebrachten Trägerstruktur; mindestens einer an der Trägerstruktur angebrachten elastischen Vorrichtung; eine Prüfmasse, die an der mindestens einen elastischen Vorrichtung aufgehängt ist und sich frei in einer x-Richtung, einer y-Richtung und einer z-Richtung bewegen kann; mindestens eine obere Elektrode, die an der mindestens einen elastischen Vorrichtung angeordnet ist; und mindestens eine untere Elektrode, die sich unterhalb der mindestens einen elastischen Vorrichtung befindet, so dass eine Anfangskapazität zwischen der mindestens einen oberen Elektrode und der mindestens einen unteren Elektrode erzeugt wird, wobei: die mindestens eine elastische Vorrichtung auf dem Substrat verankert ist; die mindestens eine elastische Vorrichtung mindestens eine mechanische Schicht und mindestens eine leitende Schicht umfasst; die mindestens eine mechanische Schicht ferner mindestens eine isolierende Schicht umfasst, die die mindestens eine leitende Schicht von der mindestens einen oberen Elektrode elektrisch isoliert; und die Antriebs-/Sensorschaltung, die Prüfmasse, die Trägerstruktur und die mindestens eine obere Elektrode und die mindestens eine untere Elektrode auf der mindestens einen Halbleiterschicht hergestellt sind.

Jayanetti V C et al. offenbaren in der Druckschrift "Design and simulation of a MEMS based dual axis capacitive accelerometer", 2015, MORATUWA ENGINEERING RESEARCH CONFERENCE (MERCON), IEEE, 7. April 2015 (2015-04-07), Seiten 194-198, XP032780691, DOI: 10.1109/MERCON.2015.7112344 einen kapazitiven MEMS-Beschleunigungssensor mit 2 Freiheitsgraden (D.O.F.). Der 2mm x 2mm x 100µm große Sensor hat einen Arbeitsbereich von bis zu 16 g und eine Bruchgrenze von 20 g. Die Bewegung einer Prüfmasse, die 74,5 % der Sensorfläche ausmacht, wird zur Erzeugung verstärkter Spannungssignale auf der Grundlage der Kapazitätstheorie mit Hilfe einer Reihe von kapazitiven Kammerelementen am Umfang des Sensors genutzt. Die Prozessvereinfachung wird durch die Verwendung eines einzigen Siliconon-Insulation-Wafers und eines minimalen Maskierungsmaterials erreicht.

Des Weiteren zeigen Kurdle D et al. in der Druckschrift "Single-crystal silicon micromirror array with polysilicon flexures", SENSORS AND ACTUATORS A: PHYSICAL ELSEVIER BV, NL, Bd. 119, Nr. 2, 13. April 2005 (2005-04-13), Seiten 559-566, XP004852199, DOI: 10.1016/J.SNA.2004.10.038 eine einkristalline Silizium-Mikrospiegelanordnung mit Polysilizium-Flexoren. Mikrospiegel-Arrays mit hoher Dichte (1296 Spiegel) werden mit einem Verfahren hergestellt, das die Vorteile der Volumen- und Oberflächenmikrobearbeitung kombiniert. Die mit dieser Technik hergestellten Arrays zeichnen sich durch optisch flache Spiegel mit einem Krümmungsradius (ROC) von ~ 1 m, eine konstante Federhomogenität (~ 6 %) über das gesamte Array und eine Ausbeute von mehr als 90 % aus. Jedes Array-Element besteht aus einer kardanischen Spiegelstruktur, die es dem Spiegel ermöglicht, sich um zwei orthogonale Achsen zu drehen, wenn an die Elektroden unter dem Spiegel eine Spannung angelegt wird. Ein trockenes Trennverfahren, kombiniert mit dem Ätzen durch den Wafer und dem Schleuderguss von Polyimid, wird als einfache, effektive Alternative zum Trocknen der Strukturen am kritischen Punkt vorgestellt. Kippwinkelmessungen für diese Bauelemente zeigen einen stabilen Winkelbereich von +-5° (mechanisch) bei 160-170 V.

Im Zuge dieser Erfindung wurde ein neuartiges, oberflächen-integriertes, mechano-optisches Mikrosensorsystem konzeptioniert, gestaltet, prototypisiert und getestet, welches die Messung piko-bis mikronewton kleiner Zugkräfte gleichzeitig in drei Dimensionen ermöglicht. In diesem System wird die Verschiebung eines Sensorelementes mit wohl definierter Struktur direkt mit einer bestimmten Kraft korreliert, indem zuvor die Kraftkonstanten für jede Raumrichtung ermittelt werden.

Das 3D-Kraftsensorsystem soll gleichzeitige quantitative dreidimensionale Kraftmessungen ausführen können und insbesondere soll es auch gleichzeitig den Zustand des Sensorelements aufzeichnen und zwar vor, während und nach der Messung.

**Gelöst** werden die Aufgabe bzw. die Aufgaben mit einer Vorrichtung zur Bestimmung von Kräften im Piko-Newton- bis Nano-Newton-Bereich gemäß Hauptanspruch. Weitere vorteilhafte Ausgestaltungen finden sich in den Unteransprüchen.

Die erfindungsgemäße Vorrichtung zur Bestimmung von Kräften im Piko-Newton- bis Nano-Newton-Bereich umfasst
- wenigstens eine Kraftmessscheibe zur Kraftmessung in drei Raumrichtungen aufweisend eine ebene, regelmäßige Anordnung von Kraftmesszellen, wobei eine Kraftmesszelle gebildet ist in einem Loch in der Kraftmessscheibe, in dem genau ein von Federn freitragend gehaltenes Flächenelement angeordnet und parallel zur Kraftmessscheibe verlaufend ausgerichtet ist, wobei jede Feder an einem ersten Ende mit dem Rand eines Lochs und an einem zweiten Ende mit dem Rand eines Flächenelements stoffschlüssig verbunden ist,
   sowie weiterhin
- Mittel zum optischen Messen der dreidimensionalen Auslenkungen der Flächenelemente der wenigstens einen Kraftmessscheibe,
und ist dadurch gekennzeichnet, dass
a. die Kraftmessscheibe, die Federn und die Flächenelemente aus demselben Material gebildet sind,
b. die Federn als längliche Mäander-Strukturen mit mehr als zwei Mäander-Perioden ausgebildet sind und
c. jedes Flächenelement von vier rotationssymmetrisch angeordneten Federn umgeben ist,
d. jedes Flächenelement unter Einwirkung einer Kraft über die Federn in drei Raumrichtungen elastisch auslenkbar ist ohne dabei wesentlich zu verkippen ("Tilt"), wobei Auslenkung und Kraft in linearer Beziehung stehen und
e. die Durchmesser der Löcher in der Kraftmessscheibe sich zu den Durchmessern der Flächenelemente etwa wie 10 : 1 verhalten.

Ferner ist es vorteilhaft die Strukturbreite der Mäander kleiner als 2 Mikrometer einzurichten.

Vorteilhaft ist es, wenn der Durchmesser der Kraftmesszellen kleiner als 200 Mikrometer eingerichtet ist.

Weiter von Vorteil ist es, wenn die Kraftmessscheibe aus einem biokompatiblen Material, aus bevorzugt Nickel-Titan-Legierung oder aus amorphem Silizium, gebildet ist.

Die Anordnung der Kraftmesszellen kann entlang zweier zueinander senkrecht stehender Achsen ausgerichtet sein.

Die länglichen Mäander-Strukturen können insbesondere entlang derselben Achsen ausgerichtet sein, die die Anordnung der Kraftmesszellen festlegen.

Vorteilhaft ist es, wenn das einer Kraftmesszelle zugeordnete Loch in der Kraftmessscheibe kreisförmig ausgebildet ist.

Die Flächenelemente auf einer vorbestimmten Flachseite können verspiegelt ausgebildet sein.

Weiter können die Flächenelemente auf der der Verspiegelung gegenüberliegenden Flachseite eine Funktionsschicht tragen.

Das Material der Kraftmessscheibe weist insbesondere ein Elastizitätsmodul aus dem Intervall von 1 bis 80 GPa auf.

Die Vorrichtung kann weiterhin ergänzend eine elektronische Übersetzungseinrichtung aufweisen, ausgebildet zur Bestimmung der auf die Flächenelemente wirkenden Kräfte aus den gemessenen Auslenkungen.

Die Erfindung sieht insbesondere die Gestaltung einer Kraftmessscheibe aus einem gegenüber den Zellen zunächst unflexiblen Material (in einer bevorzugten Ausführungsvariante mit einem Elastizitätsmodul 1 - 80 GPa) vor, welches vorstrukturiert wird, um lokale Flächenelemente für kleine Kräfte im Piko- bis Nano-Newton-Bereich sensitiv zu machen. Besonderer Wert wird daraufgelegt, dass die Auslenkung jedes Flächenelementes in drei Dimensionen dem Hook'schen Gesetz folgt, also der angreifenden Kraft direkt proportional ist. Zudem sind die einzelnen Flächenelemente, die auf der Kraftmessscheibe in einem Array angeordnet sind, voneinander mechanisch vollständig entkoppelt, da das unflexible Material zwischen den Flächenelementen keine Kräfte transportiert.

Die Flächenelemente sind als freitragende, an insbesondere Mäanderfedern aufgehängte Strukturen ausgebildet, die direkt aus dem unflexiblen Material herausgearbeitet werden. Die Kraftmessscheibe weist insofern Löcher auf, in denen die Flächenelemente elastisch gelagert sind. Feder und Flächenelement bestehen dabei insbesondere aus demselben Material wie die Kraftmessscheibe, vorzugsweise aus biokompatibler Nickel-Titan-Legierung oder aus amorphem Silizium.

Die Auslenkungen der Flächenelemente sollen von der Rückseite der Scheibe her optisch erfasst werden können, während die Zellen auf der Vorderseite aufliegen. Die optische Erfassung der dreidimensionalen Auslenkungen kann mittels Auflichtbild (x,y) und Interferenzbild (z, Phaseninformation) erfolgen. Sind die drei Federkonstanten aller Flächenelemente durch Vorkalibrierung bekannt, so können die wirkenden Kräfte ohne aufwendige Modellierung bestimmt werden. Es ist von Bedeutung, dass die Flächenelemente bei der Auslenkung möglichst nicht verkippen, weil aktive Beleuchtung zur Interferometrie benötigt wird und das reflektierte Licht zur Kamera gelangen muss. Dies wird durch bestimmte Dimensionierungsparameter erreicht.

Die Besonderheit der in der Kraftmessscheibe der Erfindung verwendeten Strukturen ist jedoch insbesondere darin zu sehen, dass ein Flächenelement durch die Mäander in derselben Ebene (unbelastet) bei Kraftbelastung in drei Richtungen linear ausgelenkt wird ohne dabei wesentlich zu verkippen ("Tilt").

Der Zustand der Sensorelemente wird insbesondere während der Messung dabei kontinuierlich mitaufgenommen.

### Ausführungsvariante:

In der Erstentwicklung eines Systemmusters wurden zunächst geeignete Sensorelemente mittels Topologieoptimierung und linear statischer Finite Elementanalyse konzipiert. Hier zeigten freistehende Sensorelemente, die aus vier Federarmen mit jeweils drei Schlaufen aufgebaut, symmetrisch angeordnet, in einem Rahmen angebracht und im Zentrum des Elementes mit einer Mittelplatte verbunden sind, die besten Eigenschaften bei lateralen und axialen Verschiebungen im Nanometerbereich. Diese Designergebnisse wurden mittels etablierter Mikromaterialbearbeitungsprozesse aus biokompatiblen Nickel-Titan (NiTi) und amorphen Silizium (a-Si)-Dünnschschichten hergestellt. Ein minimaler SensorelementDurchmesser von 45 µm bei einer Schichtdicke von 200 nm und einer Strukturgröße von 1 µm wurde erreicht. Desweiteren wurde ein Prozess entwickelt um vollständig biokompatible, Gold beschichtete Polydimethylsiloxan (PDMS) Mikrostrukturen in Membranen herzustellen. Dies legt den Ausgangspunkt für die Herstellung attraktiver, günstiger Sensorelement-Arrays mit einfach variierbaren Federkonstanten durch Variation der Polymerkomponenten. Für zukünftige genaue und quantitative Zugkraftmessungen wurden Atomkraftmikroskopie-Cantilever basierte Kalibrierungen der axialen und lateralen Sensorelement-Kraftkonsten etabliert. Zum ersten Mal wurde dabei ein diamagnetischer Levitationskraftkalibrator (D-LFC) als geeignete Kalibrierungsmethode für die Sensorelemente genutzt, dessen Genauigkeit zu 1% ermittelt wurde. Die damit bestimmten Federkonsten des 45 µm kleinen a-Si Sensorleementes betragen 0,012 N/m aus der Ebene und 4,66 N/m in der Ebene, während die Federkonstanten des 170 µm großen NiTi Sensorelemente mit 0,004 N/m axial und 0,087 N/m lateral kleinstmöglich sind.

Für eine günstige, einfache, kompakte, umfassend veränderbare und empfindliche mechanooptische Datenauslesung wurde ein Aufbau konzeptioniert und getestet, der auf einer Kombination digitaler holografischer Mikroskopie (DHM) und digitaler Bildkorrelation (DIC) basiert. Diese Auslesemethode ermöglicht die kleinstmögliche Auflösung von Verschiebungen von 200 nm. Bei Einsatz der NiTi Sensorelemente können so axiale Zugkräfte im Piko- und Nanometerbereich und lateral im Nano bis Mikronewton Bereich quantitativ gemessen werden.

Um die Adhäsionspunkte der Proben auf der Sensorelement-Mittelplatte zu kontrollieren wurde schließlich eine Technik konzeptioniert und durchgeführt, in der Ink-Jet Drucken mit der etablierten Methode der Diblock-Copolymer Mizellen Nanolithographie (BCML) kombiniert wurde. Damit wurden schnelle, genaue, simultane Mikro-und Nanogoldpunkt Drucke auf glatten, empfindlichen Bauelementoberflächen wie den Sensorelementen mit Nutzer-definierten Mikrostrukturen realisiert, die quasi hexagonale Gold Nanopartikel Muster enthalten. Durch diese Methode wird die gezielte Adhäsion und die Kontrolle der Anzahl von Adhäsionsstellen für zukünftige 3D Zellkraftverteilungsmessungen ermöglicht.

Erstmals wurde eine Kombination aus digitaler Holographie für Out-of-Plane-Verschiebungsmessungen und Bildkorrelation für In-Plane-Verschiebungsmessungen in einer Einstellung kombiniert, um Haftkräfte quantitativ in drei Dimensionen zu messen. Bei dieser vergleichsweise einfachen und kostengünstigen Variante werden die Zellkräfte über die Erfassung der Verschiebung von modifizierbaren Sensorelementen mit definierten Federkonstanten ausgelesen. Kräfte lebender Zellen können lokal auf der Zelloberfläche gleichzeitig dreidimensional erfasst werden. Mit Hilfe einer in dieser Erfindung konzipierten und getesteten Technik kann die Anzahl der Adhäsionsstellen auf der Sensorelementoberfläche über tintenstrahlbedruckte Nanodotmuster auf dem Sensorelement gesteuert werden. Auf diese Weise können umfassende mechanische Studien für Zellen verschiedener Art, wie z.B. Krebs- oder Gewebezellen, und Umweltauswirkungen auf das Zellverhalten, wie z.B. toxische Umgebungen, durchgeführt werden. Darüber hinaus können zellinspirierte Materialien direkt über die gleichen Prüfparameter bewertet werden, die auch für entsprechende Messungen an Zellen verwendet werden. Damit wäre dieses Sensorsystem nicht nur für 3D-Kraftmessungen im Submikronewton-Bereich, sondern auch für die Prüfung der Haftungsqualität neuer Biomaterialien einsetzbar.

Im Vergleich zu bisherigen Verfahren ermöglicht dieses neuartige Kraftsensorsystem eine quantitative Adhäsionskraftmessung im Piko- bis Mikrometerbereich in drei Dimensionen und zudem vollständig biokompatibel und weiter ist es modifizierbar. Dementsprechend kann der Kraftbereich in Abhängigkeit von der definierten Aufgabe durch die Wahl des Sensorelementmaterials, der Größe und Dicke variiert werden. Die Anordnung kann für zukünftige Anwendungen kompakter gestaltet werden und ist im Vergleich zu anderen Systemen kostengünstiger und einfacher in der Handhabung. Darüber hinaus kann die Leistung durch die Entwicklung zukünftiger algorithmischer Codes und durch die Reduzierung der Strukturgröße in der lithografischen Verarbeitung kontinuierlich verbessert werden. Schließlich kann die Anwendung als Add-On-Anwendung für eine Kombination mit Anordnungen wie Rasterkraftmikroskopen, für zusätzliche Messungen globaler Adhäsionskräfte oder Fluoreszenzmikroskopen zur Überwachung von Zellen während der Migration in der Draufsicht verwendet werden. Um genaue Messungen zu gewährleisten, ermöglichen die über DHM zugänglichen Pseudo-3D-Plots auch eine Auswertung des Zustands des Sensorelements.

Mit dem hergestellten NiTi-Sensorelement können Traktionskräfte von bis zu 800 pN erreicht werden. Bis zu 20 nN außerhalb der Ebene und zwischen 17 nN und 0,8 µN in der Ebene können über diesen einfachen, kostengünstigen, leicht handhabbaren und umfassend modifizierbaren Aufbau gelöst werden. In der Folge können zelluläre Kräfte und schwache Haftkräfte in dieser Einstellung wahrgenommen werden. Dies kann für Kraftcharakterisierungen wie neue biomimetische Materialien mit ausgeprägtem Haftverhalten genutzt werden. Zusätzlich werden dreidimensionale Informationen über den Zustand der Sensorelementarrays während des Abtastvorgangs über Pseudo-3D-Diagramme zur weiteren Validierung und Kontrolle der Datenzuverlässigkeit zur Verfügung gestellt.

Dieses Kraftmesssystem kann durch Entwicklungen in der Mikrofertigung, optimierte Algorithmen sowie optische Auflösungsverbesserungen weiter verbessert werden. Hier eröffnet eine weitere Verbesserung der Strukturauflösung der lithographischen Verfahren die Möglichkeit der Sensorelementreduktion und Erhöhung der Sensorarraydichte. Nach der International Roadmap of Systems and Devices können die herstellbaren Dimensionen zukünftiger Elektronikgeräte innerhalb der nächsten zehn Jahre deutlich verringert werden. Diese Entwicklungen werden die weitere Miniaturisierung der Sensorelemente vorantreiben.

Die rasante Entwicklung neuer, leistungsfähigerer Algorithmen in den Bereichen Machine Learning, Big Data Acquisition, aber auch innerhalb der Gesellschaft der Holographie und Optik wird in Zukunft eine noch schnellere Bildverarbeitung ermöglichen. Dies ermöglicht die Live-Überwachung der Sensor-Kraftmessung.

Schließlich werden weitere Verbesserungen in der Optik und der optischen Auflösung für weitere Verbesserungen in der Auflösung des neuartigen Kraftsensorsystems relevant sein. Die Erweiterung von einem Sensorelement zu einem Array von Sensorelementen kann mit Hilfe von Mikrolinsen-Arrays realisiert werden. Dieses Verfahren wird bereits in der Kamerabranche eingesetzt und könnte die Möglichkeit einer direkten Kraftverteilung bei dynamischen Ereignissen wie Zellmigration oder Zellbruch bieten.

Zukünftige genaue Kraftmessungen werden nun durch die Etablierung der momentan besten geeigneten und zugänglichen federnden Kalibrierverfahren unter Berücksichtigung der Größe des Sensorelements, der Genauigkeit der etablierten Kalibriertechniken und der Komplexität der Handhabung gewährleistet. Sie erwiesen sich als in guter Übereinstimmung mit numerisch vorhergesagten Werten, die durch die Finite-Elemente-Analyse simuliert wurden; hier können Out-of-Plane-Federkonstanten durch atomkraftmikroskopische Cantilever-Sensor-Elementmessungen mit einer Genauigkeit von etwa 20 % bestimmt werden. Für Federkonstanten in der Ebene erwies sich ein diamagnetischer Levitationskraft-Kalibrator (DLFC) als adäquates Kalibrierverfahren mit einer hohen Genauigkeit von etwa 1%. Hierdurch kann die axiale und laterale Federkonstante der verschiedenen Sensorelemente bestimmt werden.

Um die Haftung auf den Mittelscheiben der Sensorelemente für zukünftige Anwendungen zu kontrollieren, kann oder wird der Inkjetdruck verwendet, um die empfindlichen Mittelscheiben der Sensorelemente durch benutzerdefinierte Mikrotropfen zu funktionalisieren. Für die Zelltests werden definierte Zellbindungsstellen auf der Elementoberfläche benötigt, die über gleichzeitig gedruckte Gold-Nanodot-Muster steuerbar sind, die anschließend biofunktionalisiert werden. In dieser Erfindung wurde das Verfahren zur gleichzeitigen Hochdurchsatz-Mikro-Nanomusterung der Sensorelementoberflächen erarbeitet und erfolgreich getestet. Dabei wird das berührungslose, einfache, schnelle, genaue, kostengünstige, reproduzierbare und hochmodifizierbare Verfahren des Tintenstrahldrucks mit dem etablierten Gold-Nanodot-Musterverfahren der Blockcopolymer-Micell-Nanolithographie (BCML) kombiniert. Dies ermöglichte benutzerdefinierte Mikromuster von 4 x 4 Tröpfchenmatrizen mit einem getrockneten Tröpfchendurchmesser im Bereich von 70 µm auf a-SI- und NiTi-basierten Oberflächen innerhalb einer Druckzeit von ca. 16 s. Auf glatten Oberflächen sind quasi hexagonale Muster von Gold-Nanopartikeln mit einem Zwischenpartikelabstand von ca. 30 nm innerhalb einer Sekunde bedruckbar und stehen in sehr guter qualitativer Übereinstimmung mit Nanomustern, die durch Spin-Coating erzeugt wurden. Dieses hexagonale Nanomuster unterstützt die deutliche Adhäsion von Zellen auf der Oberfläche des Sensorelements. Nachfolgend werden Ausführungsbeispiele der Erfindung anhand der beiliegenden Abbildungen beschrieben, wobei diese die Erfindung erläutern sollen und nicht beschränkend für den Schutzbereich zu werten sind.

### Abbildung 1:

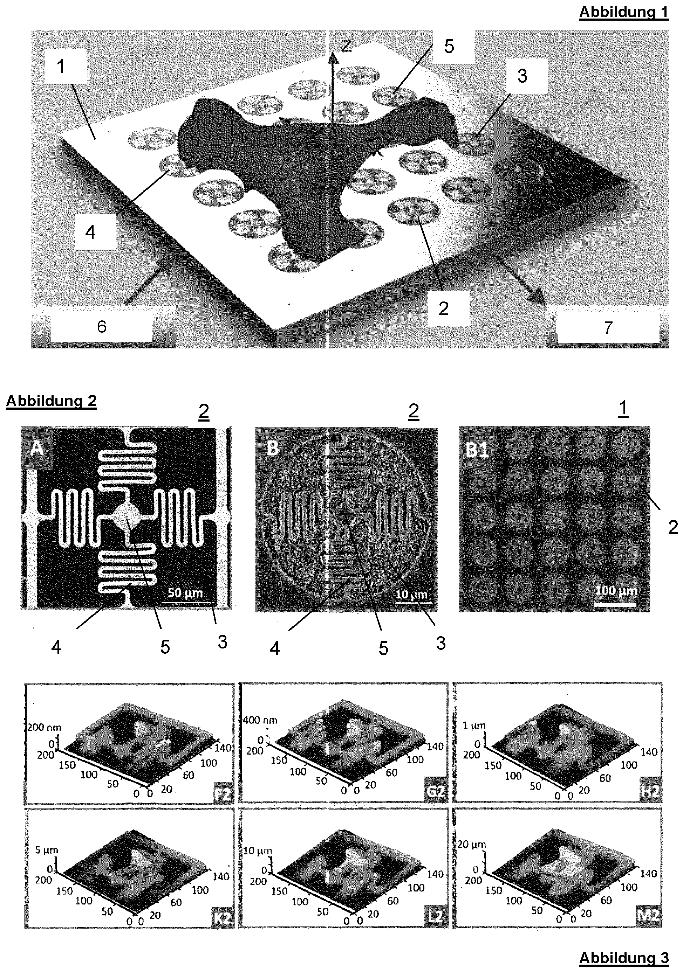

Es wurde ein Funktionsmodell eines neuartigen oberflächenintegrierten mechanisch-optischen Mikrosensorsystems gemäß Abbildung 1 konzipiert, konstruiert und getestet, welches für die gleichzeitige Erfassung von Piko- bis Nanonewton-Zugkräften in dreidimensionalen Pseudo-Dimensionen, einschließlich einer kontinuierlichen Überprüfung des Sensorelementzustands während der Messung ermöglicht. Hier wird die Verschiebung einer Kraftmesszelle 2 / eines Sensorelements mit einer gut definierten Struktur direkt mit einer bestimmten Kraft korreliert, indem die Elementfederkonstante für jede Raumrichtung bestimmt wird.

Aus diesem Grund wurden Kraftmesszellen 2 / Sensorelemente mittels Topographie und Finite-Elemente-Analyse für maximale Leistung und Empfindlichkeit entwickelt. Hier zeigt ein Element, bestehend aus vier Federn 4 / Federarmen mit drei Neigungen, symmetrisch angeordnet, an einem Rahmen befestigt und in der Mitte über ein Flächenelement 5 / eine Sensorelement-Mittelplatte verbunden, die beste Performance bei Verschiebungen innerhalb und außerhalb der Ebene (siehe Abbildung 2 A und 2 B).

Die Abbildung 1 zeigt also eine schematische Darstellung des mechanisch-optischen Mikrosensorsystems bestehend aus einer biokompatiblen Kraftmessscheibe 1 / Sensorelementanordnung, einer Laserquelle 6 mit zusätzlicher optischer Einstellung und einem Detektor 7 zum Auslesen der kraftinduzierten Sensorelementverschiebung. Hier werden die Flächenelemente 5 / Sensorelement-Mittelplatten biofunktionalisiert (zentrale Punkte in der Mitte jeder einzelnen Kraftmesszelle 2 / jedes einzelnen Sensorelements), um die Adhäsion einer Zelle (Objekt oberhalb des Sensors) an die Anordnung für 3D Zelladhäsionskraftmessungen zu steuern.

### Abbildung 2:

Die Abbildung 2 aufgeteilt in A, B, B1 zeigt freistehende Kraftmesszellen 2 / Sensorelemente, hergestellt durch Mikrobearbeitung von **A** - Nickel-Titan (NiTi) mit einer Dicke von 200 nm und einer Kantenlänge von 170 µm und **B** - amorphem Silizium (a-Si) mit einer Dicke von 200 nm und einem Mindestdurchmesser von 45 µm. **B1** - 5 x 5 a-Si Mikrosensorelementanordnung.

Basierend auf diesen Ergebnissen wurden im 5 x 5 Kraftmessscheiben 1 / Sensorelementarrays (entsprechend Abbildung 2 B1) aus vollständig biokompatiblen Materialien hergestellt. Die nasschemische Mikrobearbeitung wurde zur Herstellung von 200 nm dünnen, stabilen, freistehenden Kraftmesszellen 2 / Sensorelementen auf Nickel-Titan (NiTi)-Basis mit einer minimalen Kantenlänge von 170 µm für Folien mit einer Auflösung von 2 µm Baugröße eingesetzt (siehe Abbildung 2 A).

Für zukünftige Anwendungen mit hoher räumlicher Auflösung sind kompaktere Array-Designs durch Trockenätzmikrobearbeitung von amorphem Silizium (a-Si) herstellbar. Diese Kraftmesszellen 2 / Sensorelemente erwiesen sich als um 200 % seitenverschiebbar in ihrer ursprünglichen Federarmlänge. Eine Reduzierung der gesamten Sensorelementgröße von 70 % im Vergleich zu den NiTi-Sensorelementen auf einen minimalen Sensorelementdurchmesser von 45 µm bei einer Gerätematerialdicke von 200 nm und einer minimalen Strukturgröße von 1 µm wurde erreicht. Darüber hinaus wurde ein Plasmaätzverfahren entwickelt, um voll biokompatible, freistehende, vergoldete Polydimethylsiloxan (PDMS)-Strukturen in Membranen mit einer Kantenlänge von mindestens 1 mm herzustellen. Damit ist der Ausgangspunkt für die Herstellung attraktiver, kostengünstiger Kraftmessscheiben 1 / Sensor-Arrays mit leicht abstimmbaren Federkonstanten durch Variation der Polymerzusammensetzung und weitere Reduzierung des Bauteildurchmessers gegeben. Zusammenfassend lässt sich sagen, dass biokompatible Kraftmesszellen 2 / Sensorelemente mit unterschiedlichen mechanischen Eigenschaften für unterschiedliche Kraftmessaufgaben bereitgestellt werden können.

### Abbildung 3:

Abbildung 3 zeigt 3D-DHM-Bilder für verschiedene Verschiebungszustände des in Abbildung 2A dargestellten NiTi-Sensorelements.

Ein geeignetes, empfindliches optisches Auslesesystem wurde konzipiert und getestet. Hier wurde in ersten Messungen eine Kombination aus Off-Axis-Digital-Holographie (DHM) und Digitaler Bildkorrelation (DIC) eingesetzt, um Verschiebungen innerhalb (DIC) und außerhalb der Ebene (DHM) bis zu 200 nm für die Traktionskraftmessung aufzuzeichnen. Außerhalb der Ebene liegende Verschiebungen sind exemplarisch für ein NiTi-Sensorelement in Abbildung 3 dargestellt.

### Abbildung 4:

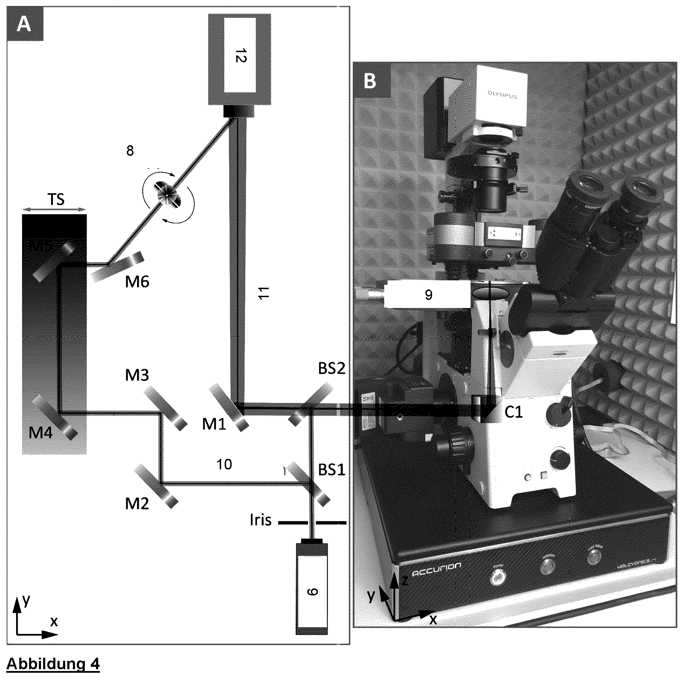

In Abbildung 4 ist eine schematische Darstellung eines DHM/DIC-Aufbaus (A), der exemplarisch als Addon-Aufbau in Kombination mit einem AFM (B) zur lokalen und globalen 3D-Kraftmessung von Zellen dient, gezeigt. BS1 und BS2 sind Strahlteiler, M1 bis M6 sind Spiegel, C1 ist ein optischer Würfel zur Kopplung des Objektivstrahls 11 im inversen Mikroskop. Der Chopper 8 wird benötigt, um den Referenzstrahl 10 zur Aufnahme laser-mikroskopischer Bilder der Messzelle 2 / des Sensorelements für die weitere digitale Bildkorrelation und die Kraftmessung in der Ebene zu blockieren.

Für zukünftige Anwendungen kann das DHM/DIC-Haftkraftsensorsystem (siehe Abbildung 4 A) als Add-On-Anwendung mit anderen Aufbauten wie Rasterkraftmikroskopen (AFM, siehe Abbildung 4 B) kombiniert werden, wie in Abbildung 4 schematisch dargestellt. Hier können Sensorelementverschiebungen durch die DHM/DIC-Einstellung von der Unterseite der oberhalb des Objektivs 9 auf dem Probenhalter des inversen Mikroskops positionierten Elemente erfasst werden, während eine an der Kraftmessscheibe 1 / Sensoranordnung haftende Zelle über den Cantilever eines darüber liegenden AFM-Kopfes manipuliert werden kann. Diese Anordnung würde z.B. eine lokale Kraftmessung über das DHM/DIC und globale Kraftmessungen von oben ermöglichen.

### Bezugszeichenliste

- 1: Kraftmessscheibe
- 2: Kraftmesszelle
- 3: Loch
- 4: Feder
- 5: Flächenelement
- 6: Laserquelle
- 7: Detektor
- 8: Chopper
- 9: Objektiv
- 10: Referenzstrahl
- 11: Objektivstrahl
- 12: Kamera
- BS1, BS2: Strahlteiler
- M1...M6: Spiegel
- C1: optischer Würfel

## Patentansprüche

1. Vorrichtung zur Bestimmung von Kräften im Piko-Newton- bis Nano-Newton-Bereich umfassend
- wenigstens eine Kraftmessscheibe (1) zur Kraftmessung in drei Raumrichtungen aufweisend eine ebene, regelmäßige Anordnung von Kraftmesszellen (2), wobei eine Kraftmesszelle gebildet ist in einem Loch (3) in der Kraftmessscheibe (1), in dem genau ein von Federn (4) freitragend gehaltenes Flächenelement (5) angeordnet und parallel zur Kraftmessscheibe (1) verlaufend ausgerichtet ist, wobei jede Feder (4) an einem ersten Ende mit dem Rand eines Lochs (3) und an einem zweiten Ende mit dem Rand eines Flächenelements (5) stoffschlüssig verbunden ist,
sowie weiterhin
- Mittel zum optischen Messen der dreidimensionalen Auslenkungen der Flächenelemente (5) der wenigstens einen Kraftmessscheibe (1),
**dadurch gekennzeichnet, dass**
a. die Kraftmessscheibe (1), die Federn (4) und die Flächenelemente (5) aus demselben Material gebildet sind,
b. die Federn (4) als längliche Mäander-Strukturen mit mehr als zwei Mäander-Perioden ausgebildet sind und
c. jedes Flächenelement (5) von vier rotationssymmetrisch angeordneten Federn (4) umgeben ist,
d. jedes Flächenelement (5) unter Einwirkung einer Kraft über die Federn (4) in drei Raumrichtungen elastisch auslenkbar ist ohne dabei wesentlich zu verkippen ("Tilt"), wobei Auslenkung und Kraft in linearer Beziehung stehen und
e. die Durchmesser der Löcher (3) in der Kraftmessscheibe (1) sich zu den Durchmessern der Flächenelemente (5) etwa wie 10 : 1 verhalten.

2. Vorrichtung nach Anspruch 1 aufweisend eine elektronische Übersetzungseinrichtung ausgebildet zur Bestimmung der auf die Flächenelemente (5) wirkenden Kräfte aus den gemessenen Auslenkungen.

## Claims

1. Device for determining forces in the pico-Newton to nano-Newton range, comprising
- at least one force measuring disc (1) for measuring force in three spatial directions featuring a flat, regular arrangement of force measuring cells (2), wherein a force measuring cell is formed in a hole (3) in the force measuring disc (1), in which exactly one surface element (5) held in a cantilevered manner by springs (4) is arranged and aligned parallel to the force measuring disc (1), each spring (4) being connected at a first end to the edge of a hole (3) and at a second end to the edge of a surface element (5) in a material-locking manner,
and further
- means for optically measuring the three-dimensional deflections of the surface elements (5) of the at least one force measuring disc (1),
**characterised in that**
a. the force measuring disc (1), the springs (4) and the surface elements (5) are made of the same material,
b. the springs (4) are designed as elongated meander structures with more than two meander periods and
c. each surface element (5) is surrounded by four rotationally symmetrical springs (4),
d. each surface element (5) can be elastically deflected in three spatial directions under the action of a force via the springs (4) without tilting significantly, whereby the deflection and the force are in a linear relationship, and
e. the diameters of the holes (3) in the force measuring disc (1) are approximately 10 : 1 in relation to the diameters of the surface elements (5).

2. Device according to claim 1, comprising an electronic translation device designed to determine the forces acting on the surface elements (5) from the measured deflections.

## Revendications

1. Dispositif pour déterminer des forces dans la plage comprise entre le pico-Newton et le nano-Newton, comprenant
- au moins un disque de mesure de force (1) pour mesurer la force dans trois directions spatiales présentant un agencement plan et régulier de cellules de mesure de force (2), une cellule de mesure de force est formée dans un trou (3) dans le disque de mesure de force (1), dans lequel exactement un élément plat (5) maintenu en porte-à-faux par des ressorts (4) est disposé et orienté parallèlement au disque de mesure de force (1), chaque ressort (4) étant relié par adhérence de matière à une première extrémité au bord d'un trou (3) et à une deuxième extrémité au bord d'un élément plat (5) par liaison de matière,
ainsi que
des moyens pour mesurer optiquement les déviations tridimensionnelles des éléments plats (5) du au moins un disque de mesure de force (1),
**caractérisé en ce que**
a. le disque de mesure de force (1), les ressorts (4) et les éléments plats (5) sont formés à partir du même matériau,
b. les ressorts (4) sont conçus comme des structures sinueuses allongées avec plus de deux périodes sinueuses et
c. chaque élément plat (5) est entouré de quatre ressorts disposés de manière symétrique en rotation (4),
d. chaque élément plat (5) peut être dévié élastiquement dans trois directions spatiales sous l'effet d'une force exercée par les ressorts (4) sans basculer de manière significative (« tilt »), la déviation et la force étant dans une relation linéaire, et
e. les diamètres des trous (3) dans le disque de mesure de force (1) sont dans un rapport d'environ 10:1 par rapport aux diamètres des éléments de surface (5).

2. Dispositif selon la revendication 1, comprenant un dispositif de conversion électronique conçu pour déterminer les forces agissant sur les éléments de surface (5) à partir des déviations mesurées.
